Europäisches Patentamt

European Patent Office

Office européen des brevets

(19)

(11) Publication number: **0 075 828**
**A1**

(12) **EUROPEAN PATENT APPLICATION**

(21) Application number: **82108677.4**

(22) Date of filing: **20.09.82**

(51) Int. Cl.³: **C 07 C 46/04**
**C 07 C 50/12, C 07 C 50/18**
**B 01 J 23/92**

(30) Priority: **28.09.81 US 306295**

(43) Date of publication of application:
**06.04.83 Bulletin 83/14**

(84) Designated Contracting States:
**AT BE CH DE FR GB IT LI LU NL SE**

(71) Applicant: **DIAMOND SHAMROCK CORPORATION**
**717 North Harwood Street**
**Dallas Texas 75201(US)**

(72) Inventor: **Mayeda, Edward A.**
**6118 Middleridge Road**
**Madison Ohio 44057(US)**

(72) Inventor: **Abrahamson, Donald W.**
**417 Hawkins Drive**
**Painesville Ohio 44077(US)**

(74) Representative: **Weickmann, Heinrich, Dipl.-Ing. et al,**
**Patentanwälte Dipl.-Ing. H. Weickmann Dipl.-Phys.Dr. K.**
**Fincke Dipl.-Ing. F.A. Weickmann Dipl.-Chem. B. Huber**
**Dr.-Ing. H. Liska Möhlstrasse 22**
**D-8000 München 86(DE)**

(54) **Oxidizing fused ring compounds to quinones with aqueous acidic solutions of cerium.**

(57) An efficient and economical process is disclosed for oxidizing fused ring hydrocarbons to quinones by means of an aqueous acidic solution of cerium. The aqueous solution employed comprises dilute nitric acid containing a total cerium content of between about 0.5 and about 1.5 gram atoms per liter, about 30% to about 70% but not less than about 0.2 gram atom per liter is in the tetravalent state. Said aqueous acidic solution can be easily recovered and electrolytically treated at commercially attractive current densities of one ampere per square inch or higher to regenerate tetravalent cerium for reuse in oxidizing additional quantities of fused ring hydrocarbon.

EP 0 075 828 A1

## OXIDIZING FUSED RING COMPOUNDS TO
## QUINONES WITH AQUEOUS ACIDIC SOLUTIONS OF CERIUM

### INTRODUCTION

This invention relates to the oxidation of fused ring compounds to quinones (e.g., naphthalene to naphthoquinone) by reaction thereof with ionic species of tetravalent cerium in acidic aqueous solution. More specifically, our invention is concerned with a process of this type wherein the reduced acidic aqueous cerium solution from said reaction is treated in an electrolytic cell to effect sufficient anodic oxidation of trivalent cerium species therein to permit reuse thereof in oxidizing more fused ring compounds to quinone form.

### BACKGROUND OF THE INVENTION

Due to early recognition of their industrial importance, e.g., in the preparation of dyestuffs, the production of condensed ring quinones such as naphthoquinone has been a field of serious investigation for a very long time. Particularly in the case of naphthoquinone, a large segment of this work has involved various attempts to oxidize the corresponding fused ring hydrocarbon precursor, i.e., naphthalene.

Unfortunately, most such oxidation techniques tend to produce a mixture of products. The selectivity of same toward a particularly desired quinone product, e.g., 1,4 naphthoquinone, is therefore a major consideration in the evaluation of alternative processes of this type. In fact, as illustrated by U.S. Patent 3,806,469, vapor phase oxidations of naphthalene generally appear to favor production of phthalic anhydride over naphthoquinones.

Even in the field of liquid phase oxidation processes where much milder reaction temperatures generally prevail, significant formation of unwanted by-products is still a common problem, representing a definite economic disadvantage and potentially serious impediment to commercial success. Thus, early attempts at using familiar liquid oxidizing agents still produced relatively poor yields of the desired quinones. See, for example, Arnold et al report in Journal of Organic Chemistry, Vol. 5, page 251, where a 20% yield of quinone was obtained by reacting naphthalene at about $80^{\circ}C$ with a mixture of hydrogen peroxide and acetic acid, as well as U.S. Patent 2,402,226 to Hyman et al, wherein the conversion to alpha-naphthoquinone by chromic acids or sulfuric acid solutions of dichromate salts was still limited to around 40% even when the reaction was conducted with the naphthalene in solution in carbon tetrachloride.

It appears that the most impressive levels of selectivity in converting fused ring compounds to quinones have thus far been attained through the use of specialized liquid oxidizing media, namely aqueous solutions, the active oxidant species of which are usually ions containing certain variable valency metallic elements which act by being reduced from a high positive valency state to a lower one, notably systems based upon metals such as cerium or chromium. In most such systems, furthermore, the oxidizing capability of the used aqueous solution can be regenerated by an electrolytic treatment which oxidizes the variable valence metal back to its higher valence state.

In some of the prior art systems of this latter type, notably in U.S. Patent 729,502 to Moest, the fused ring organic compound is suspended in an acidic aqueous electrolyte solution containing the desired metallic oxidant (e.g., cerium) in limited amounts which are clearly insufficient for directly converting all of said organic compound to quinone form, and the entire suspension is then placed in an electrolytic cell wherein the oxidation of the organic compound and the electrolytic regeneration of metallic oxidant are effected concomitantly. Although very good conversions and high electrical efficiency are claimed therein, the practical importance of same is in doubt in view of the very low current densities employed (i.e., "up to 5 amperes per square decimeter" or a maximum of about 0.3 amp per square inch). Furthermore, said patent fails to provide any specific quantitative measurements regarding quinone selectivity or net yields.

In some of the later developments in this art, oxidation of the aromatic organic compounds with said aqueous solution of metallic oxidant is carried out in a separate reaction zone with the aromatic compound dissolved in an inert, water-immiscible solvent, and the spent metallic oxidant is then regenerated for reuse by charging to an electrolytic cell the aqueous portion of the product mixture discharged from said reaction zone (which portion usually forms a separate phase when said mixture is allowed to rest in a quiescent pool).

Exemplary references disclosing this particular approach and describing specific operating conditions for same include British Patents 1,192,037 and 1,203,434 both of which were issued to Imperial Chemical Industries, Ltd. with R.A.C. Rennie named as inventor. The main difference in the organic oxidations conducted in these two British patents is that No. 1,203,434 is concerned with a broader variety of aromatic compounds than the polynuclear substrates used in No. 1,192,037. The general processing techniques employed as well as specific operating conditions are about the same in both patents. Excellent current efficiencies for regeneration of the metallic oxidant are also reported in these patents, but once again the current densities applied therein are uniformly below economically attractive levels for commercial scale operation (generally regarded as at least about 1 ampere per square inch).

## SUMMARY OF THE INVENTION

The present invention provides a very efficient and economically favorable process for oxidizing fused ring hydrocarbons to quinones by reaction with tetravalent cerium dissolved in a dilute aqueous nitric acid solution wherein the aqueous cerium solution is then recovered and subjected to electrolytic treatment to restore the tetravalent cerium content sufficiently for said solution to be reused in oxidizing additional such hydrocarbons to quinones. A major economic breakthrough for our process is the efficient accomplishment of the electrolytic regeneration step at current densities of at least one ampere per square inch. This breakthrough is made possible by observing certain critical parameters in composition of the cerium containing aqueous nitric acid solution supplied for oxidation of said fused ring hydrocarbons, including significant levels of both tetravalent and trivalent species of cerium in properly balanced ratios.

Thus, we have found that the aqueous nitric acid solution supplied for said oxidation reaction should contain a total cerium concentration of between about 0.5 and about 1.5 gram atoms per liter about 30% to about 70% of which but not less than about 0.2 gram atom per liter should be in the tetravalent state. With such balanced proportions of tetra- and tri-valent cerium in the aqueous medium supplied to said oxidation reaction, electrolytic treatment of the aqueous stream recovered from the reaction product mixture so as to restore the tetravalent cerium concentration therein to effective levels for reuse can be very efficiently carried out in a suitable electrolytic cell using current densities of between about 1 and about 3 amperes per square inch.

The oxidation reaction itself can be conducted in any suitably equipped reactor having effective means to keep the fused ring hydrocarbon (in either liquid or finely divided solid state) well dispersed in the cerium carrying, aqueous nitric acid solution. The reaction proceeds fairly readily even at room temperatures but. is, of course, accelerated by elevated temperatures. Generally, temperatures somewhere between about $10^o$ and about $110^oC$ are both practical and favorable for yielding the quinone products desired. The use of a water-immiscible, inert solvent for the fused ring hydrocarbon is often helpful in attaining an intimate dispersion of said hydrocarbon in the aqueous nitric acid solution during the oxidation reaction and assuring a uniform reaction with good yields. Carbon tetrachloride and chloroform are ideal solvents for this purpose.

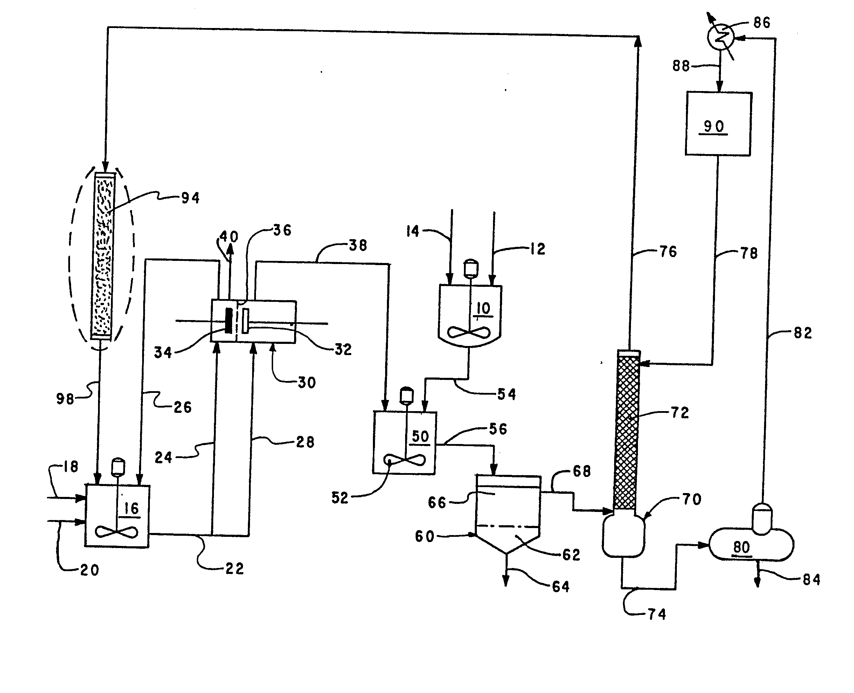

<div align="center">BRIEF DESCRIPTION OF THE DRAWING</div>

The drawing illustrates, in simplified flow sheet format, preferred embodiments wherein the major steps of the integrated process of the present invention are carried out in continuous fashion.

<div align="center">DETAILED DESCRIPTION AND PREFERRED EMBODIMENTS</div>

The fused ring, aromatic compounds of principal interest as raw materials in the present process are naphthalene and anthracene. Either of these compounds is readily oxidized to the quinone form by tetravalent cerium in aqueous acidic solution. Each tetravalent cerium participating in this reaction is reduced to the trivalent state by extracting an electron from the fused ring hydrocarbon substrate. Since a total of 6 electrons are lost in converting a molecule of the fused ring hydrocarbon to quinone form, 6 atoms of tetravalent cerium are reduced in forming one molecule of quinone. In most cases, it is desirable to react most of the fused ring hydrocarbon charged to the reaction mixture and thereby restrict the amount of unconverted material sufficiently that recovery of same is not vital to the economic operation of the process. Therefore, it is usually preferable to use about stoichiometric proportions of tetravalent cerium or a reasonable excess thereof, e.g., from about 6 to 10 gram atoms of tetravalent cerium per mol of fused ring hydrocarbon. It is also feasible to operate with much higher than stoichiometric ratio of tetravalent cerium (such as about 12 gram atoms per mol), or even at somewhat below stoichiometric (such as about 5 gram atoms per mol), with provision preferably being made in the latter case to recover and recycle unreacted hydrocarbon substrate.

As already indicated in the "SUMMARY" above, dilute aqueous nitric acid is the ideal carrier solution for the cerium oxidant in the present process. Moreover, about 0.5 to about 3 normal nitric acid solutions are the most suitable strengths for maintaining the balanced cerium concentrations which we have found to be outstandingly effective and economically advantageous in an integrated regenerative process, notable tetravalent cerium levels in the range about 0.2 to about 1.0 gram atom per liter at the start of the oxidation reaction, together with the corresponding trivalent cerium levels associated therewith as specified in the "SUMMARY" statement previously outlined herein.

The oxidation reaction can be effected with the fused ring hydrocarbon in the solid state provided it is well dispersed in the aqueous oxidant solution. This generally requires not only very energetic agitation of the dispersed system during the oxidation reaction, but also originally providing the solid hydrocarbon substrate in a special, finely divided form such as particles condensed from a steam-sublimed stream of said substrate. However, it is generally preferred to conduct said oxidation reaction with the fused ring hydrocarbon in the liquid state. This is most desirably and conveniently accomplished by predissolving said hydrocarbon in a suitable inert solvent, although naphthalene can also be dispersed in the aqueous oxidant solution in the straight molten state by intensively intermixing same at temperatures above 80°C.

Preferably, water-immiscible, highly chlorinated hydrocarbon solvents such as chloroform or carbon tetrachloride are used, and relatively low concentrations of the fused ring hydrocarbon in the resulting solutions are desirable, e.g., between about 0.05 and about 1.0 mol per liter and preferably between about 0.1 and about 0.5 mol per liter. With such organic solutions of the hydrocarbon substrate, the preferred reaction temperatures will usually not substantially exceed the atmospheric boiling point of the organic water-immiscible solvent involved, the optimum temperature range being about 30° to about 80°C.

With reaction temperatures in said optimum range and using an efficient mixing system to maintain intimate contact between fused ring hydrocarbon dissolved in a suitable solvent and sufficient aqueous oxidant solution for complete oxidation of said hydrocarbon to the desired quinone form, reaction times in the range of about 15 to about 150 minutes are usually propitious for accomplishing both good conversions and high yields. Somewhat longer reaction times (e.g., 3 hours or more may, of course, be advisable for reactions carried out at room temperatures or below).

In addition, for best overall results considering both rate of conversion of hydrocarbon substrate and the selectivity of the reaction to yield the desired quinone product, the optimum composition of the aqueous oxidant at the start of the oxidation reaction will correspond to about 1 to about 2 normal nitric acid concentration and about 0.6 to about 1.3 gram atoms of total cerium per liter, about 1/3 to about 2/3 of which is in the tetravalent state.

Having attained a mature stage in said oxidation reaction, the resulting mixture is transferred from the highly agitated reaction zone to a more stagnant zone wherein the spent aqueous oxidant solution readily separates (e.g., under the influence of gravity) from the immiscible organic solvent containing most of the organic compounds. Suitable product recovery operations such as fractional distillation, crystallization, precipitation, filtration and/or selective solvent extraction steps can be carried out on the organic phase to recover the · desired product and possibly most of the organic solvent for reuse, etc.

Meanwhile, the aqueous phase is purified (for example, by solvent extraction to remove organic contaminants) and circulated to a suitable electrolytic regeneration treatment in order to restore the tetravalent cerium content of same to an effective level for use in oxidizing further organic fused ring compounds (i.e., generally at least about 0.2 gram atom per liter and preferably between about 0.3 and about 0.9 gram atom per liter).

The electrolytic regeneration can be carried out in a conventional, direct current, electrochemical cell, provided that the inner surfaces and parts thereof which are exposed to the dilute aqueous nitric acid solution being treated are constructed of materials which are not subject to excessive corrosion thereby at the temperatures of operation. Operating temperatures of from about $20^\circ$ to about $100^\circ$C are generally suitable, with temperatures between about $30^\circ$ and about $90^\circ$C being normally preferred. The walls of the electrolytic cell can be formed of various $HNO_3$ resistant stainless steels or other alloys and passive metals known in the art, as well as more mundane metals which have been coated or lined with corrosion resistant materials such as glasses, ceramic glazes and resinous films. For the electrodes in the cell, one can choose from such materials as graphite, special stainless steels and other corrosion resistant alloys and metals, e.g., tantalum, titanium and/or the platinum group of metals. Preferably said electrolytic cell is subdivided into cathode and anode compartments by means of semipermeable membranes suitably mounted across the cell between the electrodes. Thus nitric acid resistant polymers such as polyethylenes, fluorocarbon resins and other halogenated plastics like PVC are available in the form of microporous films which permit the passage of electrically charged ions but are essentially impervious to bulk liquids. With the use of such partitioning membranes, the spacing between anodes and cathodes can be minimized so that desirable levels of current density (e.g., between about 1 and about 3 amps per square inch) can be achieved at lower voltages and good current efficiencies, thus reducing overall power costs for the electrolytic regeneration step.

In order to insure a fuller understanding of the completely integrated process, a typical operation will now be described in conjunction with the accompanying drawing, which is a simplified flow sheet illustrating preferred embodiments carried out in substantially continuous fashion. In this flow sheet, no attempt has been made to show all of the accessory equipment such as pumps and valves; and only simple somewhat generalized diagrammatic representations are presented of those particular pieces of equipment which are depicted.

In the drawing, vessel 10 represents a reservoir in which a supply of the fused ring hydrocarbon to be reacted is prepared. Thus, the fused ring compound can be introduced at 12 and a suitable solvent or liquid carrier at 14. Alternatively, at least when naphthalene is the principal fused ring compound, it can conveniently be brought to a liquid state by merely melting same in vessel 10, and liquid solvent or carrier can then be withheld if desired or at least used in reduced amounts. When highly chlorinated solvents like carbon tetrachloride or chloroform are employed to place the fused ring compound in the preferred "liquid" solution form, the concentration of the fused ring compound therein should normally be between about 0.05 and about 1 mol per liter, and preferably between about 0.1 and about 0.5 mol per liter.

Meanwhile, the initial, cerium containing, aqueous nitric acid solution is prepared in tank 16, for example, by dissolving suitable amounts of $Ce(NO_3)_3$ (cerous nitrate) added through feed line 18 in nitric acid solution introduced through line 20. In this step, the proportions of the principal components and the diluent water are carefully adjusted to provide cerium concentrations of about 0.5 to about 1.5 gram atoms per liter in the mixed solution which is about 0.5 to about 3.0 normal in $HNO_3$. Ideally, the final mixed solution will be between about 1 and about 2 normal in $HNO_3$ and will contain at least about 0.6 gram atom of total cerium per liter.

The preliminary, cerium-containing, dilute nitric acid solution prepared in the above-described manner is then circulated from tank 16 through transfer line 22 to electrolytic cell 30 for oxidative treatment to convert a substantial portion of the cerium therein to the tetravalent state. It is preferred that cell 30 be of the membrane-divided type as shown, i.e., that anode 32 and cathode 34 be separated by a substantially liquid impermeable, ion transporting, microporous partition 36, usually formed from films or sheets of microporous polymer. With this preferred (partitioned) cell structure, the flow rate of aqueous solution circulated to cell 30 through transfer line 22 desirably exceeds somewhat the flow rate at which the treated solution will be drawn off for use in oxidizing the fused ring hydrocarbon compound, with the excess being by-passed through branch line 24 to the cathode side of cell 30 and then through return line 26 directly back to holding tank 16. Meanwhile the mainstream of said aqueous solution flows through branch line 28 into the anode compartment of cell 30 operated at a current density of between about 1 and about 3 amperes per square inch under the influence of a suitable D.C. voltage impressed across the leads to electrodes 32 and 34. In addition to the anodic conversion of some trivalent cerium to the tetravalent state, the electrolysis produced simultaneously converts hydrogen ions to hydrogen gas at the cathode, said gas being released from the cathode compartment at vent 40.

The treated aqueous nitric acid solution containing at least about 0.2 gram atom of tetravalent cerium per liter is withdrawn from cell 30 through line 38 leading to reactor 50. The rate of withdrawal of said solution from cell 30 is generally controlled to provide sufficient residence time therein to raise the level of tetravalent cerium in said withdrawn solution to between about 30% and about 70% of the total cerium therein (preferably providing concentrations of between about 0.3 and about 0.9 gram atom per liter of tetravalent cerium therein).

Reactor 50 is provided with a high intensity mixer 52 and a suitable (not depicted) heat source such as a steam jacket. The fused ring hydrocarbon substrate from vessel 10 is simultaneously introduced to reactor 50 through line 54 at a rate adjusted with respect to the feed rate of oxidant solution through line 38 so that tetravalent cerium is provided in at least about the stoichiometric proportions required to convert said fused ring hydrocarbon to quinone form. If desired, multiple reactors in series can be used in place of the single reactor 50 depicted here. In any case, the combined flow rates of said reactant streams being fed to reactor 50 (or such multiple reactors in series) should be related to the capacity thereof so as to provide residence times sufficient for a relatively complete reaction to be achieved. At least 75% of the fused ring hydrocarbon should be converted and preferably the conversion of same achieved will be even higher, e.g., about 85% or more. The residence times needed will usually range between about 20 and about 120 minutes, depending mostly upon the reaction temperature. At least somewhat elevated temperatures are generally advisable, and it is often convenient when using a solvent carrier such as chloroform or carbon tetrachloride to operate reactor 50 under solvent reflux conditions (e.g., at temperatures from about 60° to about 80°C). For this purpose, reactor 50 is conveniently equipped with a suitable reflux condenser (not depicted in the present drawing).

A reaction product effluent stream from reactor 50 is passed through line 56 to at least one suitable separation device 60. The particular type of separation device 60 actually employed will depend somewhat upon the specific physical state in which the fused ring hydrocarbon substrate is being handled in the reactor 50, but will most often include some form of gravity promoted settling zone in which separate phases or layers of material will naturally tend to form. For example, if said fused ring hydrocarbon is reacted while in solution in a high density solvent such as carbon tetrachloride or chloroform, then the lower layer ·62 forming in gravity separator 60 will consist of said heavy solvent carrying most (if not all) of the organic product stream while upper layer 66 will consist largely of the aqueous nitric acid solution. In such case, the main quinone product stream would exit through bottom line 64 for suitable purification and solvent recovery steps (not illustrated in present drawing). However, if the oxidation reaction is conducted without dissolving said fused ring hydrocarbon in a solvent, then most of the organic phase in separation device 60 will be comprised of a magma of solid particles. Such a magma could collect as either an upper or lower layer in separation device 60, depending upon the relative density of the aqueous nitric acid phase associated therewith, from which layer it could of course be readily removed and recovered.

In the presently depicted, illustrative flow sheet, the lighter, upper layer 66 is the aqueous nitric acid phase which still carries in solution substantially all of the cerium, most of which is now in the reduced (trivalent) state. Thus, although unreacted tetravalent cerium could conceivable represent a significant minor fraction of the total, it is usually more advantageous to limit same to very modest levels (e.g., not over about 0.1 gram atom per liter and preferably less). Said aqueous nitric acid phase will generally also containing minor amounts of organic compounds either in suspension or solution or both. Various methods can be used to remove such organic contaminants before said aqueous solution is subjected to electrolytic treatment for regeneration of its oxidative capacity. For example, filtration can be used for removal of suspended contaminants, and most dissolved compounds can be partly removed from the aqueous phase by cooling or chilling. However, in the present illustrative flow sheet, solvent extraction of said aqueous phase is the primary purification step. Thus, as illustrated in the attached drawings, such solvent extraction is conducted on aqueous phase 66 after introducing same via line 68 into the bottom of column 70. As said aqueous phase slowly rises through "trickle-type" packed bed 72 (of contact promoting solid shapes such as Beryl saddles, glass helices or the like) in the upper part of column 70, it is intimately contacted by the higher density, water-immiscible solvent (e.g., chloroform or carbon tetrachloride) which upon being supplied to column 70 via line 78 from supply tank 90 then trickles down through said packed bed 72 (i.e., countercurrent to said aqueous phase) under the influence of gravity. The heavier, water-immiscible solvent phase is removed from the bottom of column 70 through line 74, while the thus extracted aqueous nitric acid solution exits at the top through line 76. If desired (and as illustrated in the drawing), the said solvent stream can be subjected to a distillation step in still 80 to recover a reusable solvent stream through vapor stack 82 as well as a bottom stream 84 of extracted organic compounds. The latter may include such possibilities as unreacted fused ring compounds, additional quinone products and various organic by-products (e.g., nitroquinones). The recovered solvent can be collected in cold-water condenser 86 and returned to supply tank 90 through connection 88.

Meanwhile, the extracted aqueous nitric acid exiting from column 70 through line 76 can often be recycled directly back to holding tank 16 via return line 98. However, another alternative is to include the optional illustrated feature (enclosed by the "dotted line" envelope shown in attached drawing) of inserting a bed of activated charcoal 94 in return line 98 to remove further traces of organic contaminants from the recycled aqueous stream by absorption on said charcoal. The additional use of an activated charcoal bed in this manner prevents the gradual build-up of organic contaminants in the aqueous nitric acid solution as it is recycled repeatedly through the entire process. Since such organic contaminants can deleteriously affect not only current efficiency in the electrolytic cell 30 but also the completeness and/or selectivity of the oxidation reaction conducted in reactor 50, the inclusion of such a secondary purification of the aqueous stream in the present process yields worthwhile benefits in many cases.

The following non-limiting examples are provided to illustrate the practical overall operation of our process and to demonstrate some of the special advantages obtainable in preferred embodiments of the present invention. These examples relate generally to continuous modes of operation substantially as depicted in the accompanying flow sheet, an explanatory description of which has already been given herein.

## EXAMPLE 1

Using carbon tetrachloride as the solvent, a 0.1 molar solution of naphthalene was prepared in vessel 10 and an initial, cerium-carrying, aqueous nitric acid solution containing 435 grams (1 mol) of $Ce(NO_3)_3 \cdot 6H_2O$ and 126 grams (2 mols) of $HNO_3$ per liter was made up in tank 16. Both compartments of electrolytic cell 30 (having a platinized titanium anode 32 and a directly opposed carbon cathode 34 completely separated by a liquid impervious, ion-transporting partition 36 comprising a perfluorinated hydrocarbon type of polymeric membrane) were filled with said aqueous solution. The filled cell was then activated with electrical energy of 5 to 6 volts, adjusted to provide a current density therein of about 2 amperes per square inch. After about 10 minutes operation, the tetravalent cerium concentration in the anode compartment had reached a level of slightly over 0.4 gram atom per liter.

At this point, fresh aqueous solution from tank 16 was pumped into both sides of said cell at constant flow rates set to provide an average residence time therein of about 9 minutes. As the aqueous solution displaced from the anode compartment entered the heated and well-stirred reactor 50, the introduction thereto of the naphthalene solution from vessel 10 was simultaneously initiated at a regulated flow rate (about six-tenths that of the aqueous solution, corresponding to approximately the stoichiometric proportion of naphthalene oxidizable to naphthoquinone by the tetravalent cerium). Of course, the flow rate of the reaction mixture withdrawn therefrom was substantially equal to the combined flow rates of both of the streams entering reactor 50, which provided an average residence time of about 28 minutes at the $70^{\circ}C$ temperature at which it was maintained.

The reactor effluent was first collected in a quiescent zone where it separated into an upper aqueous layer and a bottom layer of $CCl_4$ containing most of the organic compounds. The aqueous liquid (now containing about 0.05 gram atom of tetravalent cerium per liter) was then recycled to original supply tank 16 at a steady, equilibrium flow rate passing en route through a countercurrent solvent extraction column wherein it was treated with about equal volumes of liquid $CCl_4$.

After about 3 hours of steady operation, analysis of the aqueous nitric acid solution leaving the anode compartment of the electrolytic cell indicated that an average of 36% of the trivalent cerium atoms had been converted to the tetravalent state therein at an average current efficiency of about 85%. Meanwhile, analyses of the organic compounds contained in the carbon tetrachloride layer of the collected effluent from reactor 50 revealed that over 90% of the naphthalene precursor had been converted, of which the desired naphthoquinone product accounted for all but about 15%. Nitro-naphthalene (averaging a little over 5% of the naphthalene reacted) was the principal impurity clearly identified.

## EXAMPLE 2

Further runs were made with substantially the same arrangement of equipment and using solutions of the same composition as in Example 1 but at altered flow rates. When the flow rates of the aqueous nitric acid solution through the electrolytic cell were reduced by half, about 60% of the entering trivalent cerium atoms were converted to the tetravalent state in the stream passing through the anode compartment en route to reactor 50. The naphthalene solution flow rate needed to provide a stoichiometric proportion of naphthalene to $Ce^{+4}$ in said reactor thus became substantially equal to that of the entering cerium-bearing aqueous stream, resulting in an average residence time therein of about 45 minutes.

Analyses of the various solutions revealed that the current efficiency for steady state operations under said conditions averaged about 70%, while the percentages of naphthalene converted and the selectivity of same to naphthoquinone averaged about 85% and about 75%, respectively.

When the flow rates of the aqueous nitric acid solution through said cell were further reduced to only one-third those in Example 1, about 72% of the total cerium atoms was converted to the tetravalent state in the anode compartment, leaving only about 28% as $Ce^{+3}$. However, in this case, the average efficiency of the operation fell below 50%, representing a serious waste of energy.

## EXAMPLE 3

Most conditions including the composition of the starting aqueous nitric acid solution of trivalent cerium and the flow rates of same to the electrolytic cell were as outlined in Example 1. However, instead of the 0.1 molar solution of naphthalene raw material in $CCl_4$, a more concentrated solution containing 0.3 mol of naphthalene per liter was fed to reactor 50. Accordingly, the volumetric feed rate of said naphthalene solution to said reactor was reduced to about 20% of that of the entering cerium-bearing solution in order to maintain approximately a stoichiometric balance between the reactants, resulting in an average residence time therein of about 37 minutes.

This system started-up smoothly and, based upon several hours of steady state operation, gave results comparable to those in Example 1 except for a reduction in naphthalene converted from about 90% to around the 80% level.

However, when such operations were continued beyond about 10 to 12 hours, the concentration of tetravalent cerium in the effluent from the anode compartment of the electrolytic cell began to drop noticeably from the original values of about 0.4 gram atom per liter until it leveled off at about 0.3 gram atom per liter after 24 hours, indicated a reduction in current efficiency to about the 70% level and, in turn, causing a further decrease in naphthalene conversion.

It was found that these gradual losses in electrical efficiency and productivity could be obviated by more completely removing organic impurities from the cerium-containing aqueous nitric acid stream before recycling same to supply tank 16, e.g., by passing it through activated charcoal bed 94 as shown in the accompanying flow sheet drawing.

## EXAMPLE 4

The conditions of Example 3 were repeated except that the aqueous nitric acid solution was heated to about 90°C as it was being pumped to the electrolytic cell. This resulted in an increase in current efficiency to about 90%, a level which was successfully maintained for more than a day by using the activated charcoal bed to remove residual organic impurities from the aqueous solution being recycled from the product recovery area to the supply tank for retreatment.

## EXAMPLES 5A and 5B

Operating conditions were maintained as in Example 4 except that the concentration of trivalent cerium in the starting 2 normal aqueous nitric acid solution was changed first to a lower level (0.75 gram atom/liter) and secondly to a higher level (1.25 gram atoms/liters). The results of these runs are summarized in Table I.

### TABLE I

| Run No. | Total Ce Conc. (g. atom/l.) | $Ce^{+4}$ Conc. (g. atom/l.) | Current Eff. (%) | Oxid React. Res. Time (min.) | Naph. Conv. (%) | NQ[1] Select. (%) | NN[2] Select. (%) |
|---|---|---|---|---|---|---|---|
| 5A | 0.75 | 0.34 | 81 | 34 | 80 | 82 | 8 |
| 5B | 1.25 | 0.39 | 92 | 35 | 75 | 70 | 10 |

(1) Naphthoquinone

(2) Nitronaphthalene

Comparable runs under the same conditions as outlined in Table I but using initial aqueous nitric acid of either 1 or 3 normality in $HNO_3$ content as the cerium carrier gave very similar current efficiencies and about the same overall oxidation reaction results.

However, operating the electrolytic cell at a higher current density (e.g., about 2.5 amps/square inch) caused a reduction in the current efficiency realized (e.g., to about 75% under the conditions of Run 5A), while reducing the current density under such conditions to about 1.5 amps/square inch resulted in an increase in current efficiency to about 85%.

## EXAMPLE 6

A run was conducted under similar conditions to those designated in 5A above except that the carbon tetrachloride solution of naphthalene contained 0.45 mol of naphthalene per liter, and the oxidation reactor was not heated so that it operated at about 30°C. As a result, each of the naphthalene conversion and the naphthoquinone selectivity figures were lowered somewhat, namely to about 76%.

EXAMPLE 7

Another experiment was conducted using substantially the same procedures as outlined in Example 1 except that chloroform was used as the organic solvent and anthracene was used as the fused ring compound. However, in this case, the oxidation reaction was carried out at about $60^{\circ}$C and the usable capacity of the oxidation reactor was about twice as large so as to provide an average residence time of about one hour.

Results indicated that about 85% of the anthracene was converted with a selectivity to 9,10-anthraquinone of close to 90%.

In addition to the specific illustrative embodiments shown above, many other possible modifications and variations within the scope of the principles and teachings of the present invention as set forth herein will now be clear to those skilled in the art. Accordingly, the scope of patent protection provided hereby is not to be limited by any such specific embodiments but is to be fully measured by the language of the appended claims.

## WHAT IS CLAIMED IS:

1. In a process for oxidizing fused ring, aromatic organic compounds primarily to quinone form by reacting same in the liquid or solid state with an acidic aqueous solution containing tetravalent cerium as the active oxidizing reagent, the improvement which comprises:

(a) introducing said fused ring compound into a well agitated reaction zone maintained at a temperature of between about 10°C and about 110°C along with a dilute aqueous nitric acid solution containing about 0.5 to about 1.5 gram atoms of total cerium per liter dissolved therein about 30% to about 70% of which but not less than about 0.2 gram atom per liter is in the tetravalent state, the rates of introduction of said compound and said solution being such that the proportion of tetravalent cerium is not substantially less than the amount stoichiometrically required to oxidize said compound to quinone form and the residence time being sufficient to react most of said fused ring compound;

(b) subjecting the product mixture from said reaction zone to separation operations to recover the quinone product stream and remove most organics from the aqueous nitric acid solution; and

(c) circulating the thus recovered and purified nitric acid solution through at least the anode region of an electrolytic cell operated at a temperature of between about 20° and about 100°C and a current density of at least about 1 ampere per square inch until the concentration of tetravalent cerium therein has been substantially increased so that it can be effectively used in oxidizing additional amounts of fused ring compounds.

2. A process as in Claim 1 wherein the dilute nitric acid solution introduced in step (a) contains between about 0.5 and about 3 gram mols of $HNO_3$ per liter and between about 0.2 and 1.0 gram atom of tetravalent cerium per liter.

3. A process as in Claim 2 wherein the $HNO_3$ concentration is between about 1 and about 2 gram mols per liter and the tetravalent cerium concentration is between about 0.3 and about 0.9 gram atom per liter.

4.     A process as in Claim 1 wherein the fused ring compound introduced in step (a) is in solution in an inert, water-immiscible solvent.

5.     A process as in Claim 4 wherein the temperature in step (a) is between about $30^{\circ}$ and about $80^{\circ}$C.

6.     A process as in Claim 4 wherein said solvent is a highly halogenated hydrocarbon such as chloroform or carbon tetrachloride, and the concentration of the fused ring compound therein is between about 0.05 and about 1.0 gram mol per liter.

7.     A process as in Claim 6 wherein the concentration of said fused ring compound is between about 0.1 and about 0.5 gram mol per liter.

8.     A process as in Claim 1 wherein the temperature of the electrolytic cell in step (c) is between about $30^{\circ}$ and about $90^{\circ}$C.

9.     A process as in Claim 8 wherein the current density in said cell is between about 1 and about 3 amperes per square inch.

10.     A process as in Claim 1 wherein in step (a) the proportion of tetravalent cerium to fused ring compound is between about 5 and about 10 atoms per mol.

11.     A process as in Claim 10 wherein in step (b), after recovering the main quinone product stream, the aqueous nitric acid solution separated therefrom is subjected to solvent extraction to remove organic contaminants before it is returned to the electrolytic cell in step (c).

12.     A process as in Claim 10 or 11 wherein said aqueous nitric acid solution is passed through an activated charcoal bed before it is returned to the electrolytic cell in step (c).

European Patent
Office

EUROPEAN SEARCH REPORT

**0075828**
Application number

EP 82 10 8677.4

## DOCUMENTS CONSIDERED TO BE RELEVANT

| Category | Citation of document with indication, where appropriate, of relevant passages | Relevant to claim |
|---|---|---|
| Y,D | GB - A - 1 192 037 (I.C.I.) <br> * claim 12 * <br> -- | 1 |
| Y,D | GB - A - 1 203 434 (I.C.I.) <br> * claims 1, 6 * <br> -- | 1 |
| Y | Chemical Abstracts vol. 34, no. 11 <br> 10 June 1940 <br> Columbus, Ohio, USA <br> G.F. SMITH et al. "Cerate oxidimetry. Electrolytic oxidation of cerium without the use of a diaphragm cell" column 4012, no. 8 <br> & Ind. Eng. Chem. Anal. Ed. vol. 12, 1940, pages 268 to 269 <br> ---- | |

### CLASSIFICATION OF THE APPLICATION (Int. Cl. 3)

C 07 C   46/04

C 07 C   50/12

C 07 C   50/18

B 01 J   23/92

### TECHNICAL FIELDS SEARCHED (Int.Cl. 3)

B 01 J   23/92

C 07 B    3/00

C 07 C   46/04

C 07 C   50/12

C 07 C   50/18

### CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

The present search report has been drawn up for all claims

| Place of search | Date of completion of the search | Examiner |
|---|---|---|
| Berlin | 20-12-1982 | KNAACK |